# EUROPEAN PATENT APPLICATION

(11) **EP 4 382 077 A1**
(43) Date of publication of application: **12.06.2024**
(21) Application number: 22853360.0
(22) Date of filing: 29.07.2022
(51) Int. Cl.: A61F 2/00, A61B 17/06, A61B 17/00

(54) **LIFTING THREAD HAVING FLEXIBLE VARIABLE PART**

(30) Priority: 04.08.2021 KR 20210102541
(71) Applicant: Neo Dr. Inc., Wonju-si, Gangwon-do 26311 (KR)
(72) Inventor: KIM, Hyeon Ho, Wonju-si Gangwon-do 26457 (KR)
(74) Representative: Caspary, Karsten
(86) International application number: PCT/KR2022/011159
(87) International publication number: WO 2023/013982

(57) **Abstract**

The present disclosure relates to a lifting thread having a flexible variable part in which a lifted portion is firmly fixed without drooping using a normal medical thread which is relatively inflexible, a structurally stretchable variable part is formed on one part of the medical thread, and pain is not caused at a surgical site and the medical thread is prevented from breaking even if muscles undergo large movements such as those at the time of laughing or yawning, which includes catching parts, each having a first fixing protrusion and a second fixing protrusion which face each other on both sides of a fixing groove formed in an outer peripheral surface of the medical thread and a length-variable part formed in a spring shape to be capable of generating elasticity between the catching parts, thereby having structural elasticity, and in which elasticity is provided to the medical thread as the spring shape of the length-variable part increases when an external force is applied thereto.

## Description

### TECHNICAL FIELD

The present disclosure relates to a thread for an embedding procedure, and more particularly, to a lifting thread having a flexible variable part in which a lifted portion is firmly fixed without drooping using a normal medical thread which is relatively inflexible, a structurally stretchable variable part is formed on one part of the medical thread, and pain is not caused at a surgical site and the medical thread is prevented from breaking even if muscles undergo large movements such as those at the time of laughing or yawning.

### BACKGROUND

In recent years, as interest in skin care has increased, a thread-embedding therapy in which skin sagging or wrinkles are improved using medical threads has been developed and is widely used.

Medical threads inserted through a thread-embedding therapy have the effect of stimulating and pulling the surrounding skin tissues, strengthening muscles, improving skin sagging or wrinkles, and is absorbed into the treated skin over time.

Also, the threads used in such a thread-embedding therapy is usually administered through a thread inserter and such a thread inserter extends long in a forward/rearward direction and is configured to include a pipe-shaped insertion needle having a handle provided on a rear end part and a thread whose one end is inserted inside the insertion needle and the other end is exposed to the outside and extends rearward.

In the thread inserter, the thread is drawn into the human body through the insertion needle if the insertion needle is inserted into the human body and only the thread remains inside the human body if the insertion needle is withdrawn from the human body in a state in which the thread has reached a desired site inside the human body. In this way, a thread remaining inside the human body pulls and fixes the tissues inside the human body, thereby improving skin sagging and wrinkle.

On the other hand, in order to improve wrinkles or maintain skin tissue elasticity by inserting a thread into the human body in this way, the larger the diameter of the inserted thread, the greater the effect. Here, if a diameter of the insertion needle increases, for reasons such as worsening pain and causing side effects, the diameter of the insertion needle is limited and the thread inserted into the insertion needle is also used with the diameter thereof limited to a certain range.

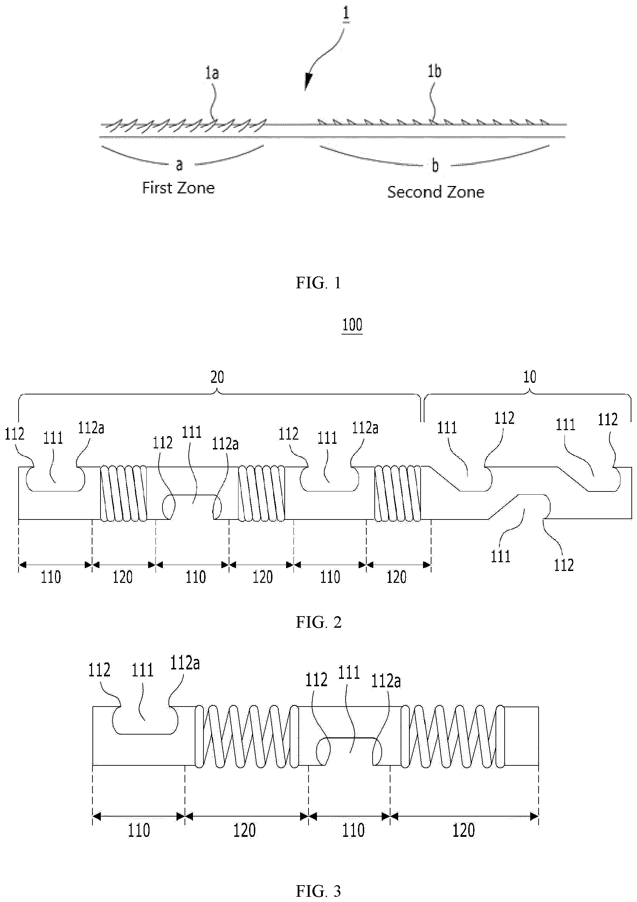

FIG. 1 shows a suture 1 for a lifting procedure having a straight shape in the related art, the suture is divided into first and second zones centering on a certain point, a plurality of protrusions 1a, 1b are formed on the periphery of the first zone a and the second zone b inclined at an angle facing each other, and the protrusion 1a of the first zone a serves as an anchor which lifts sagging skin and prevents the pulled up the tissues from sagging again.

Here, since such a suture 1 for a lifting procedure in the related only plays the role of pulling the skin under the skin, there is a problem in which the skin is continuously maintained in a rigid state and natural frowning or smiling facial expressions may not be made.

Since sutures in the related art are mostly made of non-elastic materials, when the treated muscles such as when yawning or laughing loudly move a lot, pain may be caused at the surgical site, the tissues may be torn, or in severe cases, the suture for the lifting procedure may break, reducing the lifting effect.

The present disclosure suggests a new type of suture for a lifting procedure which may effectively solve the matters of a suture for a lifting procedure in the related art.

### SUMMARY

Therefore, the present disclosure was made to solve the above matters, and the present disclosure is for the purpose of a suture for a lifting procedure in which a lifted portion does not sag due to catching parts without an elastic structure, a length-variable part capable of performing firmly lifting, and a structurally stretchable length-variable part is formed at a site having a large movement so that, even if the muscles (tissues) move significantly such as when laughing or yawing, causing of pain and thread breakage may be prevented.

Also, a suture for a lifting procedure which prevents the lifted tissues from sagging again due to the elasticity of the length-variable part and the gravity.

Furthermore, the present disclosure provides a suture for a lifting procedure which may be produced in a simple way and thus reduce production costs using injection, press, molding, laser, ultrasonic processing, or the like.

A lifting thread having a flexible variable part according to an example embodiment of a thread of the present disclosure for achieving the above matters includes catching parts, each having a first fixing protrusion and a second fixing protrusion which face each other on both sides of a fixing groove formed in an outer peripheral surface of the medical thread and a length-variable part formed in a spring shape to be capable of generating elasticity between the catching parts, thereby having structural elasticity, in which elasticity is provided to the medical thread as the spring shape of the length-variable part increases when an external force is applied thereto.

The first and second fixing protrusions are formed at acute angles with sharp ends and both sides may be formed at the same angle or may be formed at different angles.

The fixing groove may have a symmetrical structure first and second fixing protrusions on both left and right sides.

Also, as an example embodiment of the thread of the present disclosure, in the length-variable part, the medical thread is processed into a spring shape or expansion grooves are formed on both sides of the outer periphery of the medical thread alternately (in a zig-zag manner) and, when an external force is applied, a joining site between the expansion grooves increases, providing elasticity to the medical thread.

In the medical threads in which the expansion grooves are alternately formed in the length-variable part, a cover cylinder may be installed to reduce irritation or damage to tissues so that pain is prevented from occurring at the surgical site due to the movement of muscles.

The cover cylinder may be formed as a spring-type elastic cover tube.

Also, the length-variable part with a spring structure is formed between the pair of catching parts and a plurality of basic repeating units of such a length-variable part may be included.

Furthermore, in an example embodiment of the present disclosure, the medical thread is formed to be divided into a first region and a second region in a longitudinal direction, the first region includes the catching part in which the fixing groove having an acute fixing protrusion is formed on the outer periphery of the medical thread to pass through and fix the subcutaneous tissues of a person to be treated, the second region includes the length-variable part formed in a spring shape between the catching parts and having structural elasticity, and, when an external force is applied, the spring shape of the length-variable part increases, providing elasticity to the medical thread.

As described above, according to the lifting thread having the flexible variable part associated with the present disclosure, the lifted portion may be firmly fixed to the human body tissues not to sag, and even if muscles (tissues) move significantly such as when laughing or yawning, occurrence of pain can be prevented and thread breakage may be prevented by integrally forming the first region for firmly fixing the lifted portion so that it does not sag using a single medical thread, the catching parts formed so that the first fixing protrusion and the second fixing protrusion structurally face each other on both sides of the fixing groove, and the second region designed to be flexible and embedded in a site with high movement.

In addition, there is an advantage in which, even when a relatively inexpensive medical thread made of a non-elasticity material is used, the present disclosure may form a stretchable variable part relatively and simply using injection, press, molding, laser, ultrasonic processing, or the like, thereby lowering the production costs.

### BRIEF DESCRIPTION OF THE DRAWINGS

FIG. 1 is a front view showing a suture for a lifting procedure in the related art.
FIG. 2 is a front view showing a variable lifting thread for reducing pain and preventing sagging in human body tissues that corresponds to the present disclosure.
FIG. 3 is an enlarged view showing the variable lifting thread for reducing pain and preventing sagging in human body tissues according to FIG. 2.
FIG. 4 is a front view showing another example embodiment of the variable lifting thread for reducing pain and preventing sagging in human body tissues that corresponds to the present disclosure.
FIG. 5 is an enlarged view showing catching parts of the variable lifting thread for reducing pain and preventing sagging in human body tissues that corresponds to the present disclosure.
FIG. 6 is an enlarged view showing a length-variable part of the variable lifting thread for reducing pain and preventing sagging in human body tissues that corresponds to the present disclosure.
FIG. 7 is an enlarged view showing yet another example embodiment of the variable lifting thread for reducing pain in human body tissues that corresponds to the present disclosure.

### DETAILED DESCRIPTION

The present disclosure is in a best form thereof and suggests a lifting thread having a flexible variable part is configured to include catching parts, each having a first fixing protrusion and a second fixing protrusion which face each other on both sides of a fixing groove formed in an outer peripheral surface of the medical thread and a length-variable part formed in a spring shape to be capable of generating elasticity between the catching parts, thereby having structural elasticity and in which elasticity is provided to the medical thread as the spring shape of the length-variable part increases when an external force is applied thereto.

Since the present disclosure may be subjected to various changes and may have several example embodiments, specific example embodiments are illustrated in the drawings and described in detail in the detailed description. Here, this is not intended to limit the present disclosure to a specific thread form, but needs to be understood to include all changes, equivalents, or substitutes included in the idea and the technical scope of the present disclosure.

In the drawings, an example embodiment of the present disclosure is not limited to the specific form shown and is exaggerated for clarity. Although specific terms are used in this specification, they are used for the purpose of explaining the present disclosure and are not used for limiting the meaning or limiting the scope of rights of the present disclosure described in the claims.

In this specification, the expression "and/or" is used for including at least one of the constituent elements listed before and after the expression. Furthermore, the expression "connected/joined" is used for including being directly connected to other constituent elements or indirectly connected through other constituent elements. In this specification, a singular form also includes a plural form unless specifically stated in the phrase. In addition, as used in the specification, constituent elements, steps, operations, and elements referred to as the expression "includes" or "including" means the presence or the addition of one or more other constituent elements, steps, operations, and elements.

Moreover, expressions such as "first, second, and the like" are expressions used only for distinguishing a plurality of configurations and do not limit the order or other characteristics between the configurations.

In the explanation of example embodiments, the description that each layer (film), region, pattern, or structure is formed "above/on" or "below/under" a substrate, each side (film), region, pad, or pattern includes it is formed directly or via another layer. The standards for the above/on or below/under each layer are explained on the basis of the drawings.

Embodiments of the present disclosure will be described in detail below with reference to the attached drawings.

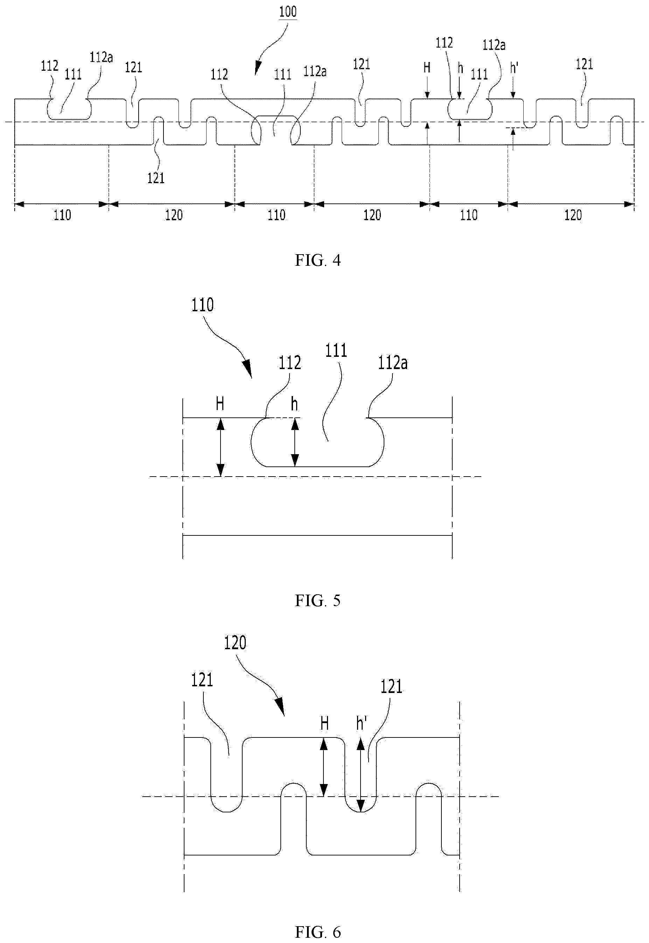

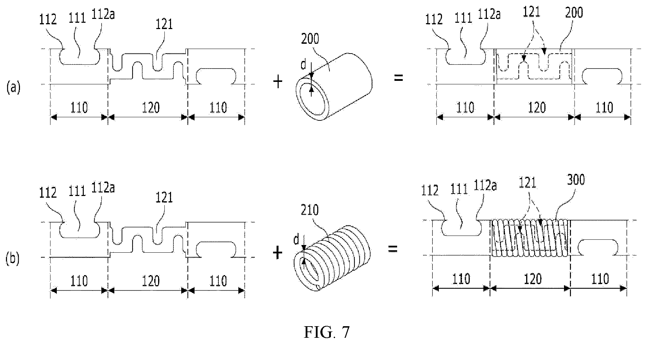

FIG. 2 is a front view showing a variable lifting thread for reducing pain and preventing sagging in human body tissues that corresponds to the present disclosure. FIG. 3 is an enlarged view showing the variable lifting thread for reducing pain and preventing sagging in human body tissues according to FIG. 2. FIG. 4 is a front view showing another example embodiment of the variable lifting thread for reducing pain and preventing sagging in human body tissues that corresponds to the present disclosure. FIG. 5 is an enlarged view showing catching parts of the variable lifting thread for reducing pain and preventing sagging in human body tissues that corresponds to the present disclosure. FIG. 6 is an enlarged view showing a length-variable part of the variable lifting thread for reducing pain and preventing sagging in human body tissues that corresponds to the present disclosure. FIG. 7 is an enlarged view showing yet another example embodiment of the variable lifting thread for reducing pain in human body tissues that corresponds to the present disclosure.

As shown in FIGS. 2 to 7, a variable lifting thread 100 for reducing pain and preventing sagging in human body tissues may include catching parts 110 and a length-variable part 120.

The catching parts each may be fixed through digging into the subcutaneous tissues of a person to be treated.

The catching parts 110 are concave fixing grooves 111 formed in a recess shape in an outer peripheral surface of a medical thread and first fixing protrusions 112 and second fixing protrusions 112a which are fixed through digging into the subcutaneous tissues may be formed at both ends of the fixing grooves 111.

Also, the first and second fixing protrusions 112, 112a are formed on both the left and right sides of the fixing grooves 111, allowing them to pass through human body tissues without being restricted due to the movement of the muscles of the operation-target person.

The first fixing protrusion 112 of the catching part 110 serves to pull up sagging tissues and the second fixing protrusion 112a is formed for the purpose of preventing tissue sagging due to the length-variable part 120 which is flexible and capable of relaxation between the catching parts 110 after the procedure.

It is preferable that such first and second fixing protrusions 112, 112a be formed in a symmetrical structure on both sides of the fixing groove 111.

The first and second fixing protrusions 112, 112a formed both the left and right sides of the fixing groove 111 are formed as inclined bevels with a sharp angle of less than 90 degrees and the first and second fixing protrusions 112, 112a may play the role of digging into human body tissues and fixing them like anchors during the procedure.

Also, the acute-angled first and second fixing protrusions 112, 112a formed on both the left and right sides of the fixing groove 111 may be produced on the outer peripheral surface of the medical thread through ultrasonic or micro-blade processing, injection, three-dimensional (3D) printing, laser processing, or the like.

The catching parts 110 may be formed into a plurality of pieces with the length-variable part and the length-variable part 120 alternately disposed upward and downward at regular intervals.

It is preferable that a depth h of the fixing groove 111 have a value smaller than that of the straight line distance H from a center of a yarn used in the production of the variable lifting thread 100 to the outer peripheral surface.

As shown in FIGS. 2 and 3, the length-variable part 120 may be formed by injection or machining the medical thread itself and processing it like a spring. That is to say, the length-variable part 120 of the thread produced in a spring shape stretches in the longitudinal direction like a spring when a tensile force is applied in the longitudinal direction.

That is to say, in the case of the length-variable part 120, as described above, due to a spring-like structure thereof, structural elasticity may be provided to a non-elastic medical thread.

Also, the length-variable part 120 with a spring structure is formed between the pair of catching parts 110 and the plurality of basic repeating units of such a length-variable part 120 may be included.

More preferably, as shown in FIG. 2, the medical thread of the present disclosure is formed to be divided into a first region 10 and a second region 20 in the longitudinal direction, the first region 10 includes the catching part 110 in which the fixing groove 111 having an acute angle of the fixing protrusion 112 is formed on the outer periphery of the medical thread to pass through and fix the subcutaneous tissues of the person to be treated, the second region 20 includes the catching parts 110 and the length-variable part 120 which is formed in a spring shape between the catching part 110 and the catching part 110 and has structural elasticity, and when an external force is applied, the spring shape of the length-variable part 120 increases and elasticity may be provided to the medical thread.

Since the fixing protrusion 112 of the first region 10 has the main purpose of fixing the tissues instead of pulling it upward, although at least one fixing protrusion 112 needs to be formed in a direction in which it faces the second region, the fixing protrusion 112 may be produced with the pair of fixing protrusions facing each other in the fixing groove 111.

In another example embodiment of the present disclosure, as shown in FIG. 4, the length-variable part 120 may be formed between the catching part 110 and the catching part 110 and a plurality of expansion grooves 121 may be formed in a zig-zag manner on both sides to provide elasticity of the variable lifting thread 100.

The expansion grooves 121 may be formed in a "U" shape or a "V" shape.

That is to say, the plurality of expansion grooves 121 are formed in a zig-zag manner between the length-variable part 120 and the catching part 110, and when an external force is applied, the connection between the expansion groove 121 and the expansion groove 121 stretches like a spring, structural elasticity is provided.

It is preferable that a depth h' of the expansion groove 121 formed in the length-variable part 120 be a value larger than that of the straight line distance H from the center of the yarn used in the production of the variable lifting thread 100 to the outer peripheral surface.

That is to say, in the case of the length-variable parts 120, as explained above, a plurality of "U"-shaped or "V"-shaped bends or grooves are formed in a form in which they are alternately disposed in a leftward/rightward direction so that the length-variable parts 120 are alternately disposed in the leftward/rightward direction or an upward/downward direction in the longitudinal direction, and due to the structure formed using this alternately-disposed structure, structural elasticity may be provided to a non-elasticity medical thread.

Also, the length-variable part 120 in a form in which the expansion grooves 121 in a "U" or "V" shape are alternately disposed in the leftward/rightward direction is formed between the pair of catching parts 110 and a plurality of basic repeating units of such a length-variable part 120 may be included.

When the skin of the person to be treated stretches by performing bending or pulling the groove site in the case of laughing or yawning, the structure shown in FIG. 4 may cause pain by providing irritation or damaging the tissues due to relative movement between the bending of the thread or the groove portion and the subcutaneous tissues.

In yet another example embodiment of the present disclosure to resolve this, as shown in FIG. 7, it is preferable that the cover cylinder 200 be installed on the outer periphery of the length-variable part 120.

As shown in FIG. 7, if the cover cylinder 200 is installed on the outer periphery of the length-variable part 120, even when the bending or groove site is pulled and stretches, the bending or groove portion of the thread stretches inside the cover cylinder 200 instead of in direct contact with the subcutaneous tissues, preventing irritation or damage to the tissues.

The cover cylinder 200 used herein is processed into a cylinder shape using a raw material usually used in producing a medical composite yarn.

The cover cylinder 200 may be formed as an elastic cover tube 210 in the form of a tension spring using a wound elasticity thread. Such an elastic cover tube 210 may be produced in the form of a tension spring (cylinder shape) with elasticity by tightly winding the medical thread in a spiral shape around a cylinder (for example, insertion needle) of a certain diameter and heating it using a heating device. As such a heating device, an oven, a hot dryer, a hair dryer, or the like may be used. At this time, it is preferable that a heating temperature be within the range of 60 to 99% of a melting point of the medical thread. Even when the internal cylinder is removed, the heat-treated medical thread created in this way has enhanced elasticity so that the elastic cover tube 210 which is tightly wound in a spiral shape is maintained without loosening like a tension spring.

At this time, it is preferable that radii of the cover cylinder 200 and the elastic cover tube 210 be formed to be equal to or smaller than radii H of the variable lifting thread 100. For this purpose, it is necessary that, at the time of producing the length-variable part 120 of the medical thread for the lifting procedure, a radius of the length-variable part 120 is reduced by an amount corresponding to a thickness d of the cover cylinder 200 or the elastic cover tube 210. That is to say, it is preferable that a distance from the center of the medical thread to the outer peripheral surface of the length-variable part 120 be formed as a straight line distance H from the center of the medical thread to the outer peripheral surface of the catching part 110 excluding a thickness d of the cover cylinder 200 or the elastic cover tube 210.

Even when the bending or groove site is pulled and stretches using the cover cylinder 200 or the elastic cover tube 210 covered around the outer periphery of the length-variable part 120, the bending or groove portion of the thread stretches inside the cover cylinder 200 instead of in direct contact with the subcutaneous tissues, preventing irritation or damage to the tissues, thereby preventing pain at the surgical site.

An action state according to the variable lifting thread for reducing pain and preventing sagging in the human body tissues of the present disclosure that corresponds to the present disclosure having the structure configured as described above is as follows.

By forming an integrated structure in which the length-variable part 120 which is formed like a spring structure is located between the catching part 110 and the catching part 110, even when the medical thread which does not have elasticity is used, after being embedded in the subcutaneous tissues of the person to be treated, it can naturally relax or contract in accordance with the movement of muscles.

Also, natural relaxation and contraction is possible in accordance with the movement of skin muscles by locating the catching parts 110 formed spaced apart from each other on the outer peripheral surface of the variable lifting thread 100 and the length-variable part 120 in which the spring structure is formed between the catching part 110 and the catching part 110 and repeating the basic repeating units including these a predetermined number of times. In addition, the unique effect of being able to contract and relax effectively while being stably fixed is achieved by forming the catching parts 110 to have an integrated structure instead of a separate joining structure in which the variable lifting thread 100 is formed.

Furthermore, the variable lifting thread 100 that corresponds to the present disclosure maintains a constant joining force with the skin through the catching parts 110 and may have an elastic restoring force due to the structural elasticity of the length-variable part 120 which is formed integrally with the catching parts 110 and continues.

Along with this, the first fixing protrusion 112 serves to pull upward sagging tissues by forming the first and second fixing protrusions 112, 112a at acute angles on both the left and right sides of the fixing grooves 111 of the catching parts 110, respectively, and the second fixing protrusion 112a is formed for the purpose of preventing tissue sagging due to the length-variable part 120 and gravity between the catching part 110 and the catching part 110 after the procedure.

As explained above, the present disclosure is not limited to the specific preferred thread example described above, of course, a person with ordinary knowledge in the technical field to which the invention pertains may make various modifications without departing from the gist of the present disclosure asserted in the claims, and such changes are with the scope of the claims.

## Claims

1. A lifting thread having a flexible variable part, comprising:
catching parts, each having a first fixing protrusion and a second fixing protrusion which face each other on both sides of a fixing groove formed in an outer peripheral surface of a medical thread; and
a length-variable part formed in a spring shape to be capable of generating elasticity between the catching parts, thereby having structural elasticity,
wherein elasticity is provided to the medical thread as the spring shape of the length-variable part increases when an external force is applied thereto.

2. The lifting thread having a flexible variable part of claim 1, wherein the first fixing protrusion and the second fixing protrusion are formed at acute angles with sharp ends.

3. The lifting thread having a flexible variable part of claim 1, wherein the fixing groove has a symmetrical structure with the first fixing protrusion and the second fixing protrusion on both left and right sides, respectively.

4. The lifting thread having a flexible variable part of claim 1, wherein the length-variable pat is formed by processing the medical thread into a spring shape so that elasticity is provided to the medical thread by stretching when an external force is applied.

5. The lifting thread having a flexible variable part of claim 1, wherein the length-variable part has expansion grooves which are alternately disposed (in a zig-zag manner) on both sides of the outer periphery of the medical thread and a joining site between the expansion grooves expands so that elasticity is provided to the medical thread when an external force is applied.

6. The lifting thread having a flexible variable part of claim 5, wherein a cover cylinder is installed on the outer periphery of the length-variable pat to reduce stimulation or damage to tissues so that pain is not caused at a surgical site due to movement of muscles.

7. The lifting thread having a flexible variable part of claim 6, wherein the cover cylinder is a spring-shaped elastic cover tube.

8. The lifting thread having a flexible variable part of claim 1, wherein the catching parts are formed to be alternately disposed upward and downward between the length-variable parts at regular intervals and a plurality of basic repeating units of the length-variable part and the catching part are included.

9. The lifting thread having a flexible variable part of claim 1, wherein the medical thread is formed to be divided into a first region and a second region in a longitudinal direction, the first region includes the catching part in which a fixing groove with an acute fixing protrusion is formed on the outer periphery of the medical thread to pass through and fix subcutaneous tissues of a person to be treated, the second region includes a length-variable part formed in a spring shape between the catching part and having structural elasticity, and the spring shape of the length-variable part increases when an external force is applied, providing elasticity to the medical thread.
